# EUROPEAN PATENT APPLICATION

(11) **EP 4 446 345 A1**
(43) Date of publication of application: **16.10.2024**
(21) Application number: 22903548.0
(22) Date of filing: 08.12.2022
(51) Int. Cl.: C07K 16/46, C07K 16/18, C07K 16/28, C07K 16/34, C07K 16/00, A61K 38/00, A61K 38/17

(54) **FC POLYPEPTIDE HAVING ALTERED GLYCOSYLATION MODIFICATION**

(30) Priority: 09.12.2021 CN 202111500682
(71) Applicant: Shanghai Bao Pharmaceuticals Co., Ltd., Shanghai 201908 (CN)
(72) Inventor: LIU, Yanjun, Shanghai 201908 (CN); WANG, Zheng, Shanghai 201908 (CN); XU, Yunxia, Shanghai 201908 (CN); LV, Baojie, Shanghai 201908 (CN)
(74) Representative: Bayramoglu et al.
(86) International application number: PCT/CN2022/137399
(87) International publication number: WO 2023/104128

(57) **Abstract**

An Fc variant containing a 2,6-sialylated glycosylation modification. The Fc variant has an increased affinity for human FcRn and an increased 2,6-sialylation level. The Fc variant can reduce the level of IgG in the blood, and 2,6-sialic acid thereof has an anti-inflammatory effect.

## Description

### Technical Field

The present invention relates to the field of biomedicine, specifically to an Fc variant containing a sialic acid modification.

### Background Art

Intravenous human immunoglobulin (IVIG) formulations, consisting of IgG antibodies from the serums of thousands of donors, were originally used as IgG replacement therapy for immunocompromised patients. Since the discovery over 30 years ago that IVIG therapy can relieve immune thrombocytopenia, the use of IVIG formulations has been expanded to a variety of autoimmune and inflammatory diseases. Despite being an effective clinical therapy, IVIG suffers from deficiencies such as affecting the renal function in patients by clinical doses of up to 1-2 g/kg, allergic reactions due to IgA impurities, potential viral infections, etc. Thus, there is a need for improved means of treating autoimmune and inflammatory diseases, such as replacement with recombinant products with equivalent efficacy.

In recent years, with the study of the mechanism of IVIG, including the occupation of FcRn site to accelerate the metabolism of pathological IgG, small amount of 2,6-sialic acid to exert anti-inflammatory effect and so on, there have been some substitutes that can exert a single function. For example, the mutant Abdegs, which increases the affinity for FcRn, has been used to decrease IgG levels in serum (Vaccaro, C. et al. (2005). "Engineering the Fc region of immunoglobulin G to modulate in vivo antibody levels." Nat Biotechnol 23(10): 1283-1288). CN107847586A discloses that the administration of an effective amount of a sialic acid-modified polypeptide to a subject can regulate the level of Treg cells in the subject so as to achieve the effect of inhibiting inflammation. However, these products replace only part of the functionality of IVIG. Therefore, there is a need for an alternative IVIG product that addresses the problems of high protein burden, inconvenience to use, infection risk, IgA allergic reactions, and limited accessibility, reduces the level of pathogenic IgG in blood, and has anti-inflammatory effects.

### Summary of the Invention

The present invention solves the above technical problems in the prior art by means of the following technical solutions:
1. A Fc variant containing a 2,6-sialylated glycosylation modification, the variant having an increased affinity for FcRn compared to a wild-type IgG.
2. The Fc variant of item 1, wherein the variant has an increased affinity for FcRn under neutral and/or weakly acidic conditions, preferably pH 6.5-7.4 for neutral conditions, preferably pH 5.0-6.5 for weakly acidic conditions.
3. The Fc variant of item 1 or 2, wherein the Fc variant is obtained by mutation based on a wild-type IgG selected from the group consisting of IgG1, IgG2, IgG3, and IgG4, preferably the Fc variant is obtained by mutation based on the wild type IgG1 or an allotype thereof.
4. The Fc variant of any of one of items 1-3, wherein compared to a wild type Fc fragment of a wild type IgG1, the Fc variant comprises a mutation (EU numbering) selected from the group consisting of M252Y/S254T/T256E/H433K/N434F, M252Y/S254T/T256E, N434A, N434W, N434Y, M428L/N434S, T250Q/M428L, T307Q/N434A, M252Y/V308P/N434Y, T307P/L309Q/Q311R, H285D/T307Q/A378V, L309D/Q311H/N434S, M252Y/T256D, and T256D/T307Q, T256D/T307W.
5. The Fc variant of any of one of items 1-4, wherein compared to a wild type Fc fragment of a wild type IgG1, the Fc variant comprises a mutation (EU numbering) selected from the group consisting of V264A, Δ446G, Δ447K, V262E, ΔE293, ΔE294, V264E, F241A, F243A, and Y300A.
6. The Fc variant of any of one of items 1-5, wherein the Fc variant has an N-glycan attached at its glycosylation site, wherein the N-glycan is highly sialylated, preferably the glycosylation site is N297 (EU numbering).
7. The Fc variant of item 6, wherein the N-glycan has a degree of 2,6-sialylation higher than 20%, preferably higher than 25%, preferably higher than 30%, preferably higher than 35%, preferably higher than 40%, preferably higher than 45%, preferably higher than 50%, preferably higher than 55%, preferably higher than 60%, preferably higher than 65%, preferably higher than 70%, preferably higher than 80%, preferably higher than 85%, preferably higher than 90%, more preferably higher than 95%.
8. The Fc variant of item 6, wherein per mole of Fc variant has a degree of 2,6-sialylation higher than 0.1 mol, higher than 0.2 mol, preferably higher than 0.3 mol, preferably higher than 0.4 mol, preferably higher than 0.5 mol, preferably higher than 0.6 mol, preferably higher than 0.7 mol, preferably higher than 0.8 mol, preferably higher than 0.9 mol, preferably higher than 1.0 mol, preferably higher than 1.1 mol, preferably higher than 1.2 mol, preferably higher than 1.3 mol, preferably higher than 1.4 mol, preferably higher than 1.5 mol, preferably higher than 1.6 mol, preferably higher than 1.7 mol, preferably higher than 1.8 mol, preferably higher than 1.9 mol, more preferably higher than 2.0 mol.
9. The Fc variant of item 6, wherein more than 40% of the N-glycan comprises one to four 2,6-sialic acid modifications at the end of the sugar chain, and less than 60% glycosyls of the N-glycan are in the form of G0, G1, G0F, G1F, G2, or G2F, where G0-G2 separately represents glycosyls that have been glycosylated by 0-2 galactose, and F represents glycosyls that have been glycosylated by fucose.
10. The Fc variant of any of one of items 1-9, wherein the 2,6-sialic acid is N-acetylneuraminic acid (NANA), N-glycolylneuraminic acid (NGNA) or a mixture thereof, preferably wherein the 2,6-sialic acid is NANA.
11. The Fc variant of item 10, wherein the Fc variant does not comprise an alpha-galactose modification.
12. The Fc variant of any one of items 1-11, wherein the Fc variant has a fucose content of less than 50%, preferably less than 10%.
13. A protein comprising the Fc variant of any one of items 1-12 and an optional element beneficial to the expression of the Fc variant; preferably, the protein contains a signal peptide at the N-terminus of the Fc variant and/or a histidine tag at the C-terminus of the Fc variant; more preferably, the protein is connected to a secretion signal sequence at the N-terminus of the Fc variant and, a methionine is connected to the N-terminus of the secretion signal sequence; more preferably, the protein comprises or consists of, from the N-terminus to the C-terminus: methionine, secretion signal sequence, and the Fc variant.
14. A composition or kit comprising the Fc variant of any of items 1-12 or the protein of item 13.
15. A method of preparing an Fc variant of any one of items 1-12, the method comprising the steps of:
   (a) mutating a wild-type Fc fragment of a wild-type IgG1 to obtain a Fc variant, wherein the sequence of the wild-type Fc fragment is shown in SEQ ID NO: 12; and,
   (b) treating the Fc variant with β-1,4-galactosyltransferase (β1,4-GT) and/or α-2,6-sialyltransferase (α2,6-ST), preferably the β1,4-GT and/or α2,6-ST are from mammalian, more preferably from human.
16. The method of item 15, wherein the mutation in step (a) is selected from the group consisting of M252Y/S254T/T256E/H433K/N434F, V264A, Δ446G, Δ447K, V262E, T307P/L309Q/Q311R, V264E, ΔE293, ΔE294, M252Y/S254T/T256E, N434A, N434W, N434Y, F241A, F243A, M428L/N434S, L309D/Q311H/N434S, M252Y/T256D, T250Q/M428L, T307Q/N434A, M252Y/V308P/N434Y, H285D/T307Q/A378V, T256D/T307Q, T256D/T307W, and Y300A.
17. An isolated nucleic acid encoding the Fc variant of any one of items 1-12 or the protein of item 13.
18. A vector comprising the nucleic acid of item 17.
19. A host cell comprising the nucleic acid of item 17 or the vector of item 18.
20. A method of preparing the Fc variant of any one of items 1-12, the method comprising the steps of:
   (i) culturing the host cell of item 19 in a culture medium under conditions that allow the expression of the Fc variant; and,
   (ii) optionally, purifying the Fc variant from cultured cells or the culture medium.
21. Use of the Fc variant of any of one of items 1-12, the protein of item 13, or the composition or kit of item 14 in the manufacture of a drug for reducing the level of IgG in the blood.
22. Use of the Fc variant of any of one of items 1-12, the protein of item 13 or the composition or kit of item 14 in the manufacture of a drug for the treatment of an antibody-mediated disorder, preferably the antibody-mediated disorder is an autoimmune disease or hyperglobulinemia.
23. The use of item 22, wherein the autoimmune disease is selected from: Addison's disease, alopecia areata, ankylosing spondylitis, antiphospholipid syndrome, aplastic anemia, anti-GBM glomerulonephritis, anti-NMDAR encephalitis, antiphospholipidemic syndrome, autoimmune gastritis, autoimmune hearing loss, autoimmune hemolytic anemia, autoimmune hepatitis, autoimmune hypoparathyroidism, autoimmune hypophysitis, autoimmune inner ear disease, autoimmune lymphoproliferative syndrome, autoimmune myocarditis, autoimmune oophoritis, autoimmune bullous skin disease, autoimmune orchitis, autoimmune polyendocrinopathy, Behcet's disease, bullous pemphigoid, cardiomyopathy, inflammatory demyelinating polyneuropathy, acute motor axonal neuropathy, Churg-Strauss syndrome, abdominal diseases, autoimmune urticaria, Crohn's disease, CREST syndrome, celiac disease, Degos's disease, anti-neutrophil cytoplasmic antibody associated vascular inflammation, autoimmune neutropenia, acquired epidermolysis bullosa, essential mixed cryoglobulinemia, giant cell arteritis, glomerulonephritis, Goodpasture's syndrome, Graves' disease, Guillain-Barre syndrome, Hashimoto's thyroiditis, primary immunologic thrombocytopenic purpura, inflammatory bowel disease, Kawasaki disease, Ménière's syndrome, mixed connective tissue disease, Mooren ulcer, rheumatoid arthritis, multiple sclerosis, myasthenia gravis, stiffman syndrome, complete congenital heart block, acquired epidermolysis bullosa, pemphigus foliaceus, pemphigus vulgaris, pernicious anemia, polyarteritis nodosa, types of polyglandular autoimmune syndrome, primary biliary cirrhosis, types of polyglandular autoimmune syndrome, types of polyglandular autoimmune syndrome, polymyositis/dermatomyositis, psoriasis, psoriatic arthritis, Raynaud's syndrome, Reiter's syndrome, rheumatic heart disease, hemophilia-acquired FVIII deficiency, Lambert-Eaton myasthenia syndrome, multiple myeloma, sarcoidosis, collagen thesaurismosis, Sjogren's syndrome, subacute thyroiditis, sympathetic ophthalmia, systemic lupus erythematosus, Takayasu's arteritis, Sjögren's syndrome, type I diabetes, vitiligo, Vogt-Koyanagi-Harada syndrome or Wegener's granulomatosis, acute asthma or chronic asthma, organ transplant rejection, primary progressive multiple sclerosis, systemic sclerosis, serological disease, and immune complex hypersensitivity; wherein the immune complex is an immune complex formed by an anti-drug antibody and a drug.
   preferably, the autoimmune disease is selected from the group consisting of anti-glomerular basement membrane antibody disease (anti-GBM antibody disease), pathogenic IgG antibody-mediated thrombotic thrombocytopenia, Guillain-Barre syndrome (GBS), autoimmune encephalitis (AE), neuromyelitis optica lineage disease (NMOSD), atypical hemolytic uremic syndrome (aHUS), antibody-mediated rejection after organ transplantation (AMR), myasthenia gravis (MG); wherein the pathogenic IgG antibody mediated thrombotic thrombocytopenia is preferably thrombotic thrombocytopenic purpura (TTP), heparin induced thrombocytopenia (HIT), vaccine induced immune thrombotic thrombocytopenia (VITT).
24. The use of any one of items 22, wherein the antibody-mediated disorder is treatable using intravenous immunoglobulin (IVIG), subcutaneous immunoglobulin (SCIG), plasmapheresis, and/or immunoadsorption.
25. The use of item 22, wherein the hyperglobulin is produced by a leukocyte selected from the group consisting of B cells and abnormal B cells; the globulin comprises a gamma globulin; the hyperglobulinemia is selected from the group consisting of primary monoclonal gammaglobulinemia, connective tissue disease, liver disease, infectious disease, sarcoidosis, myasthenia gravis, Hodgkin's disease, Behcet's disease, nephritis, allergic purpura, immune (or idiopathic) thrombocytopenia, malignant monoclonal gammaglobulinemia (such as multiple myeloma, heavy chain disease, malignant lymphoma, chronic lymphocytic leukemia, macroglobulinemia, etc.), secondary monoclonal gammaglobulinemia (such as non-lymphoreticular tumor, monocytic leukemia, cryoglobulinemia, etc.), benign M-proteinemia, monoclonal gammopathy of unknown significance (MGUS), Waldenstrom's macroglobulinemia, AL-type amyloidosis, solitary plasmacytoma (bone or extraosseous), POMES syndrome, reactive plasmacytosis, osteolytic lesions of metastatic cancer, plasmoblastic lymphoma, and monoclonal immunoglobulin-associated renal damage (MGRS); preferrably, the hyperglobulinemia is multiple myeloma.
26. Use of the Fc variant of any of one of items 1-12, the protein of item 13, or the composition or kit of item 14 in the manufacture of a drug for preventing and/or treating autoantibody-mediated organ rejection after solid organ transplantation in a subject.
27. The use of item 26, wherein the organ is selected from the group consisting of the kidney, pancreatic islet, pancreas, heart, liver, lung, or small intestine.
28. Use of the Fc variant of any of one of items 1-12, the protein of item 13, or the composition or kit of item 14 in the manufacture of a drug for gene therapy.
29. Use of the Fc variant of any of one of items 1-12, the protein of item 13 or the composition or kit of item 14 in the preparation of a drug for the clearance of a neutralizing antibody of a pre-existing antiviral vector in vivo prior to a viral vector-based gene therapy.
30. Use of the Fc variant of any of one of items 1-12, the protein of item 13, or the composition or kit of item 14 in the manufacture of a drug for the clearance of autoantibodies in a subject to render an Fc-containing agent more therapeutically effective.
31. Use of the Fc variant of any of one of items 1-12, the protein of item 13, or the composition or kit of item 14 in the manufacture of a drug for reducing the serum level of an Fc-containing agent in a subject to whom the Fc-containing agent has been administered.
32. The use of item 30 or 31, wherein the Fc-containing agent is a therapeutic or diagnostic agent, preferably the Fc-containing agent is an antibody, Fc fusion protein antibody drug conjugate.
33. The use of any one of items 21-32, wherein the Fc variant is simultaneously or sequentially administered to the subject along with another therapeutic agent, wherein the other therapeutic agent is an anti-inflammatory agent, a leukocyte depleting agent, or a B-cell depleting agent; the B-cell depleting agent is preferably used as an antibody for tumor treatment, more preferably the antibody specifically binds to CD10, CD19, CD20, CD21, CD22, CD23, CD24, CD37, CD53, CD70, CD72, CD74, CD75, CD77, CD79a, CD79b, CD80, CD81, CD82, CD83, CD84, CD3, CD11a, CD14, CD25, CD28, CD30, CD33, CD36, CD38, CD40, CD44, CD52, CD55, CD59, CD56, CD103, CD134, CD137, CD138, CD152, CD3, IL-1β, IL-2R, IL-6, IL-12, IL-23, C5, BAFF, BLyS, BCMA, CXCR-4, ICAM-1, SLAMF7/CS1, TNFα, IgE, CD85, or CD86.
34. The use of item 33, wherein the additional therapeutic agent is selected from the group consisting of rituximab, daclizumab, basiliximab, muromonab-CD3, infliximab, adalimumab, omalizumab, efalizumab, natalizumab, tocilizumab, eculizumab, golimumab, canakinumab, ustekinumab, belimumab, daratumumab, Isatuximab, or a combination thereof.

The details of one or more embodiments of the present invention are set forth in the description below. The other features, objectives, and advantages of the present invention will be apparent in the specification and claims.

### Brief Description of the Drawings

FIG. 1 shows the effects of different variants of the present invention (m1, m4, and m6) on the disease scores in an experimental autoimmune encephalomyelitis (EAE) mouse model, with PBS (phosphate buffered saline) as a negative control and IVIG as a positive control. A lower disease score represents a higher degree of health.
FIG. 2 shows the effects of different variants of the present invention (m1, m4, and m6) on the Treg cell proportion (CD25⁺foxp3⁺ / CD3⁺CD4⁺ × 100%) in an EAE mouse model, with PBS as a negative control and IVIG as a positive control. The higher the proportion of Treg cells, the better the anti-inflammatory efficacy is.

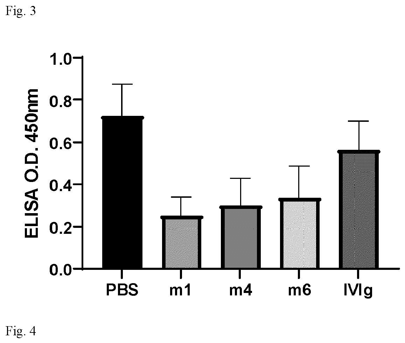

FIG. 3 shows the effects of different mutants of the present invention (m1, m4, and m6) on the level of IgG in the blood of an EAE mouse model, with PBS as a negative control and IVIG as a positive control.
FIG. 4 shows the effects of different mutants of the present invention (m1, m4, and m6) on the content of specific anti-MOG antibody in the blood of an EAE mouse model, with PBS as a negative control and IVIG as a positive control.

### Detailed Description of the Invention

In view of the shortcomings of the prior art, it is an object of the present invention to provide an Fc variant having an anti-inflammatory effect, while reducing the level of pathological IgG in the blood, and a method for preparing the Fc variant.

Specifically, the present invention relates to an Fc variant containing a 2,6-sialic acid modification. The Fc variant comprises more than 20% of 2,6-sialic acid modification. At the same time, the inventors have unexpectedly discovered that the Fc variant with superimposed sialic acid modification mutation increases FcRn affinity, has an increased level of sialic acid modification relative to the one with solitary mutation, while not affecting FcRn affinity. In addition, the Fc variant has an extended blood half-life, while having a function of lowering the level of IgG in the blood and anti-inflammation.

### 1. Definitions

The term "Fc fragment" or "Fc region" refers to the constant region of a full-length immunoglobulin excluding the first immunoglobulin constant region domain. The Fc fragment of IgG comprises CH2, CH3 domains, and a lower hinge region between CH1 and CH2. The Fc region of IgG1 is comprised of the CH2-CH3 lower hinge, i.e. the portion from amino acid C226 to the carboxy terminus, numbered according to the EU numbering or the Kabat equivalent. Similar domains of other IgG subclasses can be determined by comparing the amino acid sequences of heavy chains or heavy chain fragments of IgG subclasses with the amino acid sequences of human IgG1. As used herein, "EU numbering" or "EU numbering or Kabat equivalent" refers to the numbering of the amino acid residues of human IgG antibody.

The Fc fragment used in accordance with the present invention may also comprises a portion of the upper hinge region upstream of position 226 (EU numbering). In this case, the Fc fragment of human IgG1 comprising a portion of the region between positions 216 and 226 is preferably used. In this case, the "Fc fragment of human IgG1" refers to a portion from amino acids 216, 217, 218, 219, 220, 221, 222, 223, 224, or 225 to the carboxyl terminus. The term "Fc fragment" also refers to a single-chain Fc fragment, which is a single-chain Fc fragment obtained by gene fusion of two Fc monomers linked by a polypeptide linker. Single-chain Fc fragments naturally fold into functional dimeric Fc regions.

The term "wild-type Fc fragment" refers to a reference polypeptide in the wild-type Fc region or a variant optionally containing mutations other than those considered. The term "wild-type" or "WT" herein refers to an amino acid sequence or nucleotide sequence found in nature, i.e. it is of natural origin (including allelic variation) and has not been intentionally modified by molecular biological techniques such as mutagenesis For example, the "wild-type Fc region" specifically refers to the IgG1 Fc region with SEQ ID NO: 13 (G1m1, 17 isoform) and IgG1 Fc region with SEQ ID NO: 12 (G1m3, isoform).

The term "Fc variant" refers to an Fc fragment resulting from one or more amino acid substitutions, additions, or deletions in the Fc region. Fc variants may comprise other regions, including but not limited to Fab, scFv, VHH, etc.

The term "half-life" refers to the amount of time that an Fc fragment, once present in the serum of a patient to whom it is administered, is eliminated in half from the circulation or from other tissues.

The term "Fey receptor" or "FcyR" refers to an IgG type immunoglobulin receptor of CD64 (FcyRI), CD32 (FcyRII), and CD16 (FcyRIII), particularly the five expressed receptors (FcyRIa, FcyRIIa, FcyRIIb, FcyRIIIa, FcyRIIIb). Except for human FcyRIIb (which is a receptor that inhibits immune cell activation), they are all effector cell activation receptors (Muta T et al. Nature, 1994, 368: 70-73).

The term "FcRn" refers to a nascent Fc receptor. It refers to a protein that binds to the Fc region of the IgG antibody and is at least partially encoded by the FcRn gene. The FcRn can be derived from any organism, including, but not limited to, humans, mice, rats, rabbits, and monkeys. As is known in the art, a functional FcRn protein comprises two polypeptides, which are commonly referred to as a heavy chain and a light chain. The light chain is β-2-microglobulin and the heavy chain is encoded by the FcRn gene. Unless otherwise indicated herein, an FcRn or FcRn protein refers to a complex of an FcRn heavy chain with β-2-microglobulin. Exemplary FcRn molecules include human FcRn encoded by the FCGRT gene set forth in RefSeq NM_004107.

The term "effector cell" refers to any cell bearing an Fc receptor, such as a lymphocyte, a monocyte, a neutrophil, a natural killer (NK) cell, an eosinophil, a basophil, a mast cell, a dendritic cell, a Langerhans cell, and a platelet.

The terms "N-glycan", "N-linked glycan", "glycoprotein", "glycosylation", and "saccharide form" refer to N-linked oligosaccharides, e.g. oligosaccharides linked to an asparagine residue of a polypeptide via an asparagine-N-acetylglucosamine linkage. The primary sugars found on glycoproteins are glucose, galactose, mannose, fucose, N-acetylgalactosamine (GalNAc), N-acetylglucosamine (GlcNAc), and sialic acid (SA, including NANA, NGNA, and derivatives and analogs thereof, including acetylated NANA or acetylated NGNA). In sugar-engineered Pichia, sialic acid is only N-acetylneuraminic acid (NANA) (Hamilton et al., Science 313 (5792): 1441-1443(2006)). N-glycans have a common pentasaccharide core, i.e. Man3GlcNAc2, where "Man" refers to mannose, "Glc" refers to glucose, "NAc" refers to N-acetyl, and GlcNAc refers to N-acetylglucosamine. N-glycans differ in the number of branches (antennae) comprised by peripheral sugars (e.g. GlcNAc, galactose, fucose, and sialic acid) added to the Man3GlcNAc2 ("Man3") core structure, also referred to as "trimannose cores", "pentasaccharide cores" or "oligomannose cores". N-glycans are classified according to their branching components (e.g. high mannose, complexes, or hybrids). An N-glycan terminus can be modified with up to four sialic acids.

The term "G0" refers to a complex bidirectional antennal oligosaccharide (GlcNAc₂Man₃GlcNAc₂) without galactose or fucose; "G1" refers to a complex bidirectional antennal oligosaccharide (GalGlcNAc₂Man₃GlcNAc₂) without fucose and containing one galactose residue; "G2" refers to a complex bidirectional antennal oligosaccharide (Gal₂GlcNAc₂Man₃GlcNAc₂) without fucose and containing two galactose residues; "G0F" refers to a complex bidirectional antennal oligosaccharide (GlcNAc₂Man₃GlcNAc₂F) containing core fucose and without galactose; "G1F" refers to a complex bidirectional antennal oligosaccharide (GalGlcNAc₂Man₃GlcNAc₂F) containing one core fucose and one galactose residue; and "G2F" refers to a complex bidirectional antennal oligosaccharide (Gal₂GlcNAc₂Man₃GlcNAc₂F) containing core fucose and two galactose residues.

The term "hybrid" N-glycan refers to an N-glycan having at least one GlcNAc at the end of the 1,3-mannose arm of the trimannose core and zero or more than one mannose on 1 position of 1,3-mannose. The 6 mannose arms of the trimannose core.

The term "level of sialic acid modification" refers to the total number of the sialic acid modifications on N-glycan terminal of the Fc variant and/or its proportion to the total Fc variant. For example, each Fc variant molecule has an average of 0.1, 0.2, 0.5, 1, 2, and 4 sialic acid modifications; Alternatively, the N-glycan of the Fc variant has a 2,6-sialylation degree higher than 20%, 25%, 30%, 35%, 40%, 45%, 50%, 55%, 60%, 65%, 70%, 75%, 80%, 85%, 90%, 95%, preferably higher than 65%. The sialic acid may be modified at the time of protein expression or it may be modified in vitro in a solution in which enzymes such as sialyltransferase are added. A high level of modification means that each Fc variant molecule has on average a high number of sialic acids at the N-glycan end of each Fc variant molecule.

The term "highly sialylated" refers to the addition of one or more sialic acid groups to the N-glycan of the Fc region. The addition of one or more sialic acid groups can be carried out by enzymatic reactions, by cellular reactions, or by targeted mutagenesis of the Fc targeting one or more amino acids involved in Fc sialylation. An N-glycan terminus can be modified with up to four sialic acids. Highly sialylated specifically means that the N-glycans of the Fc variant have higher than 20% and at least 1 sialic acid modification or an average of at least 0.2 sialic acid modifications per Fc variant molecule.

IgG is the major serum immunoglobulin in blood. It is a glycoprotein consisting of two identical heavy chains and two light chains, which in turn consist of a variable region and a constant region. IgG comprises a single, N-linked glycan at position Asn297 of the CH2 domain of each of its two heavy chains. The covalently linked, complex carbohydrate is composed of a core, double-branched penta-polysaccharide containing N-acetylglucosamine (GlcNAc) and mannose.

The term "vector" or "expression vector" used herein refers to a vector used according to the present invention as a vehicle for introducing a desired gene into a cell and expressing the desired gene in the cell. One skilled in the art will recognize that such vectors can be readily selected from plasmids, phages, viruses, and retroviruses. In general, vectors suitable for the present invention will contain selection markers, suitable restriction sites to facilitate the cloning of the desired gene, and the ability to enter and/or replicate in eukaryotic or prokaryotic cells.

A variety of expression vector systems may be used for the purposes of the present invention. For example, one class of vectors utilizes DNA elements derived from animal viruses such as bovine papilloma virus, polyoma virus, adenovirus, vaccinia virus, baculovirus, retrovirus (RSV, MMTV, or MOMLV), or SV40 virus. Others involve the use of polycistronic systems with internal ribosome binding sites. In addition, cells that have integrated DNA into their chromosomes can be selected by introducing one or more biomarkers that allow for the selection of transfected host cells. The markers may confer prototrophy to an auxotrophic host, confer biocide resistance (e.g. antibiotics), or resistance to heavy metals (e.g. copper). The selectable marker gene may be directly linked to the DNA sequence to be expressed or introduced into the same cell by co-transformation. The optimal synthesis of mRNA may also require other elements. These elements may include signal sequences, splicing signals, and transcriptional promoter, enhancer, and termination signals.

More generally, once the vector or DNA sequence encoding the Fc variant has been prepared, the expression vector may be introduced into a suitable host cell. That is, it can transform host cells. The introduction of the plasmid into the host cell can be accomplished by a variety of techniques known to those skilled in the art. These techniques include but are not limited to, transfection (including electrophoresis and electroporation), protoplast fusion, calcium phosphate precipitation, cell fusion with enveloped DNA, microinjection, and infection with whole viruses. More preferably, the plasmid is introduced into the host by electroporation. The transformed cells are cultured under conditions suitable for production of the Fc variant and the expression of the Fc variant is determined. Exemplary assay techniques include enzyme-linked immunosorbent assay (ELISA), radioimmunoassay (RIA), fluorescence activated cell sorter analysis (FACS), immunohistochemistry, and the like.

As used herein, the term "transformation" shall be used in a broad sense to refer to the introduction of DNA into a recipient host cell, thereby changing the genotype and thereby causing a change in the recipient cell.

Similarly, a "host cell" refers to a cell transformed with a vector constructed using recombinant DNA technology and encoding at least one heterologous gene. In describing methods for isolating polypeptides from recombinant hosts, the terms "cell" and "cell culture" are used interchangeably to refer to the source of the Fc variant, unless specifically indicated otherwise. In other words, recovery of an Fc variant from a "cell" can mean recovery from a settled whole cell, or from a cell culture containing both medium and suspended cells.

### 2. Fc variants of the present invention

The present invention relates to an Fc variant containing glycosylation modification of 2,6-sialylation, composition, and use thereof. The Fc variant has increased affinity for at least one Fc receptor as compared to the wild-type IgG, wherein at least one Fc receptor is selected from the group consisting of FcRn, Fcy receptor, and complement C1q, preferably the Fc receptor is human FcRn.

In an embodiment, the variant has increased affinity for human FcRn under neutral conditions, preferably the neutral conditions are pH 6.5-7.4. In an embodiment, the variant has an increased affinity for FcRn under weakly acidic conditions, preferably pH 5.0-6.5. In an embodiment, the Fc variant is obtained by mutation based on a wild-type IgG selected from the group consisting of IgG1, IgG2, IgG3, and IgG4, preferably the Fc variant is obtained by mutation based on the wild type IgG1.

In an embodiment, compared to a wild type Fc fragment of a wild type IgG1, the Fc variant comprises a mutation (EU numbering) selected from the group consisting of N434A, N434W, N434Y, M252Y/S254T/T256E, M428L/N434S, M252Y/S254T/T256E/H433K/N434F, T250Q/M428L, T307Q/N434A, M252Y/V308P/N434Y, T307P/L309Q/Q311R, H285D/T307Q/A378V, L309D/Q311H/N434S, M252Y/T256D, T256D/T307Q, T256D/T307W.

In an embodiment of the disclosure, the wild-type Fc fragment is as shown in SEQ ID NO: 12. In some embodiments described herein, amino acid substitutions may be made to the constant region of an antibody. For example, different IgG1 allotypes, such as the well-known G1m17 allotype, G1m3 allotype, or G1m1 allotype, or combinations thereof.

Other Fc variants used in the present invention may further include, but are not limited to, the mutations described by Dall'Acqua et al. (WF, D. A. et al., 2002). Journal of immunology (Baltimore, Md. : 1950) 169 (9): 5171-5180), mutations described by Shan et al. (Shan, L. et al., (2016). PLoS One 11(8): e0160345.), mutations described by Lee et al., (Lee, C. H. et al., (2019). Nat Commun 10(1): 5031.), mutations described by Mackness et al., (Mackness, B. C. et al., (2019). MAbs 11 (7): 1276-1288.), mutations described by Christophe et al., (Dumet Christophe, Pottier Jérémy, Gouilleux-Gruart Valérie et al., MAbs, 2019, 11: 1341-1350).

The Fc variant that can be used for the present invention may further include additional mutations, wherein the mutation includes a mutation that enhances the affinity for Fc and Fc γ, preferably, S239D/I322E/A330L, S239D/I322E, I322E, I322E/A330L, K326W/E333S, or a sugar modification, preferably a fucose free modification. The Fc variant of the present invention may be a monomer, a dimer, and a multimer. The Fc variant that can be used for the present invention may further include additional mutations, which include but are not limited to mutations described by Wang et al (Wang Xinhua, Mathieu Mary, Brezski Randall J, (2018), Protein Cell, 9 (1), 63-73. doi:10.1007/s13238-017-0473-8).

### Glycosylation modification of Fc variant

Glycosylation modifications of Fc are involved in antibody function. Such glycosylation modification includes, but is not limited to: mannosylation, fucosylation, galactosylation, and sialylation. Among them, the sialylated modification may have various structures, including 2, 3-linked sialic acid modification, 2,6-linked sialic acid modification, 2,8-linked sialic acid modification, etc. wherein only the 2,6-linked sialic acid modification has an anti-inflammatory function. Studies have shown that 2,6-linked sialic acid modification is one of the mechanisms of IVIG's anti-inflammation (Li, D. et al., (2021). Theranostics 11(11): 5430-5446).

The 2,6-linked sialic acid modification of the Fc can be enhanced by mutation of the amino acid sequence. The Fc variants of the present invention have enhanced 2,6-linked sialic acid modification as compared to the wild-type Fc fragment of the wild-type IgG1. In an embodiment, compared to a wild type Fc fragment of a wild type IgG1, an Fc variant of the present invention comprises a mutation (EU numbering) selected from the group consisting of V264A, Δ446G, Δ447K, V262E, ΔE293, ΔE294, V264E, F241A, F243A, Y300A.

In an embodiment of the disclosure, the wild-type Fc fragment is as shown in SEQ ID NO: 12. In some embodiments described herein, amino acid substitutions may be made to the constant region of an antibody. For example, different IgG1 allotypes, such as the well-known G1m17 allotype, G1m3 allotype, or G1m1 allotype, or combinations thereof.

In an embodiment, an Fc variant of the present invention has an N-glycan at its glycosylation site, wherein the N-glycan has a biantennary and/or triantennary and/or tetraantennary glycan structure, with short chains, that is it is highly sialylated. Preferably, the N-glycan has a biantennary glycan structure. Preferably, the glycosylation site is Asn297 (EU numbering).

In an embodiment, higher than 40%, preferably higher than 40%, of the N-glycans of the Fc variants of the present invention comprise one to four 2,6-sialic acid modifications at the end of the sugar chain, and less than 60%, preferably less than 50%, of the N-glycans of the Fc variants of the present invention are glycosyls in the forms of G0, G1, G0F, G1F, G2, or G2F. Wherein: G0-G2 represents 0-2 galactosylated glycosyls and F represents fucosylated glycosyls, respectively.

In an embodiment, the N-glycan has a degree of 2,6-sialylation higher than 20%, preferably higher than 25%, preferably higher than 30%, preferably higher than 35%, preferably higher than 40%, preferably higher than 45%, preferably higher than 50%, preferably higher than 55%, preferably higher than 60%, preferably higher than 65%, preferably higher than 70%, preferably higher than 80%, preferably higher than 85%, preferably higher than 90%, more preferably higher than 95%. In an embodiment, per mole of Fc variant of the present invention has a degree of 2,6-sialylation higher than 0.1 mol, higher than 0.2 mol, preferably higher than 0.3 mol, preferably higher than 0.4 mol, preferably higher than 0.5 mol, preferably higher than 0.6 mol, preferably higher than 0.7 mol, preferably higher than 0.8 mol, preferably higher than 0.9 mol, preferably higher than 1.0 mol, preferably higher than 1.1 mol, preferably higher than 1.2 mol, preferably higher than 1.3 mol per mole, preferably higher than 1.4 mol, preferably higher than 1.5 mol, preferably higher than 1.6 mol, preferably higher than 1.7 mol, preferably higher than 1.8 mol, preferably higher than 1.9 mol, more preferably higher than 2.0 mol.

In an embodiment, the 2,6-sialic acid is N-acetylneuraminic acid (NANA), N-glycolylneuraminic acid (NGNA), or a mixture thereof. In an embodiment, the 2,6-sialic acid is NANA. In an embodiment, wherein the Fc variant does not comprise an alpha-galactose modification. In an embodiment, wherein the Fc variant does not comprise NGNA and alpha-galactose modification.

In an embodiment, the N-glycan of the Fc variant of the present invention has a fucose content of less than 50%, preferably less than 40%, preferably less than 30%, preferably less than 20%, preferably less than 10%.

In an embodiment, the present invention further comprises an isolated binding molecule comprising an Fc variant of the present invention, wherein the binding molecule is a recombinant peptide comprising: (i) a binding domain capable of binding to a target molecule on or associated with a cell, and (ii) an Fc domain having amino acid sequences of CH2 and CH3 constant domains of a human IgG1 heavy chain, characterized in that the binding molecule is capable of binding to a target molecule on a target cell. The binding molecule can be e.g. a full-length IgG antibody, Fc fusion proteins, etc.

### 3. Preparation method of Fc variant of the present invention

In an embodiment, the present invention provides a method of enhancing 2,6-sialic acid modification of an Fc or variant thereof, the method comprising treating the Fc or variant thereof with a galactosyltransferase and/or a sialyltransferase to enhance the 2,6-sialic acid modification. In an embodiment, the Fc is modified with both enzymes via an in vitro modification pathway and/or an in vivo modification pathway. In an embodiment, the in vitro modification pathway includes: subjecting galactosyltransferases and/or sialyltransferases to be reacted with an Fc protein in a buffer to increase terminal sialic acid modification. In an embodiment, the in vivo modification pathway includes: transferring the gene for galactosyltransferase and/or sialyltransferase, and the Fc gene into the cell, and the Fc is modified with sialic acid upon expression.

The methods described herein include the use of galactosyltransferases and/or sialyltransferases, such as β-1,4-galactosyltransferases (e.g. β 1,4-galactosyltransferase 1 encoded by the B4GALT1 gene), such as an α-2,6-sialyltransferase (e.g., ST6Gal-1). Many ST6Gal sialyltransferases are known in the art and commercially available (see, e.g., Takashima, Biosci.Biotechnol.Biochem.72: 1155-1167 (2008); Weinstein et al., J.Biol.Chem.262: 17735-17743 (1987)). β-1,4-galactosyltransferase 1 is a key enzyme for the transfer of galactose to the terminal N-acetylglucosamine (GlcNAc). ST6Gal-1 catalyzes the transfer of sialic acid from a sialic acid donor (e.g. cytidine 5'-monophosphate-N-acetylneuraminic acid) through an α-2,6 linkage to the terminal galactose residue of the glycan.

In an embodiment, the present invention provides a method for preparing an Fc variant of the present invention, the method comprising: (a) mutating a wild-type Fc fragment of a wild-type IgG1, the sequence of which is as shown in SEQ ID NO: 12, to obtain a corresponding Fc variant; and (b) treating the Fc variant by using β-1,4-galactosyltransferase and/or α-2,6-sialyltransferase. In an embodiment, the β1,4-GT and/or α2,6-ST are from mammalian, more preferably from human.

In an embodiment, the mutation in step (a) is selected from the group consisting of M252Y/S254T/T256E/H433K/N434F, V264A, Δ446G, Δ447K, V262E, T307P/L309Q/Q311R, V264E, ΔE293, ΔE294, M252Y/S254T/T256E, N434A, N434W, N434Y, F241A, F243A, M428L/N434S, L309D/Q311H/N434S, M252Y/T256D, T250Q/M428L, T307Q/N434A, M252Y/V308P/N434Y, H285D/T307Q/A378V, T256D/T307Q, T256D/T307W, Y300A.

In an embodiment, the Fc variant in step (a) is obtained by:
(i) culturing host cells containing nucleic acid encoding the Fc variant of the present invention in a culture medium under conditions that allow the expression of the Fc variant of the present invention; and
(ii) optionally, purifying the Fc variant of the present invention from cultured cells or the culture medium of cells.

In an embodiment, the host cell does not produce immunogenic NGNA and alpha-galactose.

In an embodiment, the present invention provides an isolated nucleic acid encoding an Fc variant of the present invention; a vector comprising the nucleic acid; and a host cell comprising the nucleic acid and/or vector. Polynucleotides encoding the Fc variants disclosed herein are typically inserted into expression vectors for introduction into host cells that can be used to produce desired amounts of the claimed Fc variants, i.e. the Fc variants of the present invention. Thus, in certain aspects, the present invention provides expression vectors comprising the polynucleotides disclosed herein, as well as host cells comprising these expression vectors and polynucleotides.

In an embodiment, a nucleic acid encoding an Fc variant described herein is co-expressed in a host cell with one or more β-1,4-galactosyltransferases and/or α-2,6-sialyltransferases, and the enzymes sialylates the Fc variant described herein.

In some embodiments, the Fc variant is expressed in a host cell, and the host cell endogenously expresses or recombinantly expresses β-1,4-galactosyltransferase and/or α-2,6-sialyltransferase. Additionally or alternatively, the host cell is cultured under conditions that increase the activity of β-1,4-galactosyltransferase and/or α-2,6-sialyltransferase in the cell, thereby producing a glycoprotein having an Fc region with sialylated branched glycans as described herein, e.g. by adding Mn²⁺ to the culture medium.

In some embodiments, the Fc variants described herein are sialylated by contacting a purified β-1,4-galactosyltransferase and/or α-2,6-sialyltransferase with an Fc variant comprising those described herein. In an embodiment, the host cell line used to express an Fc variant of the present invention is derived from a mammal; one of skill in the art can determine the particular host cell best suited for expressing the desired gene product. Exemplary host cell lines include, but are not limited to, DG44 and DUXB11 (Chinese hamster ovary cell line, DHFR⁻), SP2/0 (mouse myeloma), 293 (human kidney). In an embodiment, the cell line has altered glycosylation, e.g. afucosylation, of an Fc variant expressed therefrom (e.g. PER. C6 or CHO cell lines with FUT8 knockout). In an embodiment, NS0 cells can be used. CHO cells are particularly preferred. Host cell lines are generally available from the commercial services American Tissue Culture Collection or from the public literature.

In vitro production allows scale-up to obtain large numbers of the Fc variants of the present invention. Techniques for mammalian cell culture are known in the art and include homogeneous suspension culture, e.g. in a bioreactor, or immobilized or entrapped cell culture, e.g. in a hollow fiber, microcapsule, or agarose microbead. If necessary and/or desired, the solution of the Fc variant may be purified by conventional purification methods (e.g. gel filtration, affinity chromatography, ion exchange chromatography, hydrophobic chromatography, etc.).

Genes encoding Fc variants of the present invention can also be expressed in non-mammalian cells (e.g. yeast or plant cells). The most commonly used eukaryotic microorganisms are Saccharomyces cerevisiae or common baker's yeast, but a variety of other strains are commonly available.

In addition to cell-based expression systems, cell-free or chemical synthesis methods can be used to generate Fc variants. In certain embodiments, Fc variants are produced by in vitro chemical synthesis.

The present invention also relates to a protein containing the Fc variant of the present invention.

In some embodiments, the protein comprises an Fc variant of the present invention and elements conducive to the expression of the variant. In some embodiments, the protein comprises a signal peptide at the N-terminus of the variant. In some embodiments, the protein is connected to a secretion signal sequence at the N-terminus of the variant and, a methionine is connected to the N-terminus of the secretion signal sequence; and/or there is a histidine tag connected to the C-terminus of the variant. In some embodiments, the protein comprises or consists of from the N-terminus to the C-terminus: methionine, a secretion signal sequence, and the variants.

### 4. Composition or kit comprising the Fc variant of the present invention

The present invention relates to a composition or kit comprising the Fc variant of the present invention or a nucleic acid encoding the same.

In a specific embodiment, the composition or kit of the present invention further comprises a pharmaceutically acceptable carrier or excipient.

The pharmaceutical carrier may be a liquid and the pharmaceutical composition may be in the form of a solution. The liquid carrier is used in preparing solutions, suspensions, emulsions, syrups, elixirs, and pressurized compositions. The active ingredient can be dissolved or suspended in a pharmaceutically acceptable liquid carrier such as water, an organic solvent, a mixture of both or pharmaceutically acceptable oils or fats.

The pharmaceutical composition may be formulated for parenteral administration (e.g. intravenous or intramuscular) by bolus injection. The composition may take such forms as a suspension, solution, or emulsion in an oily or aqueous carrier, and may comprises formulation agents such as a suspending, stabilizing, and/or dispersing agent. Alternatively, the active ingredient may be in the form of a powder for constitution with a suitable carrier, e.g. pyrogen-free water.

In a specific embodiment, the composition of the present invention further comprises an antibody, a small molecule targeted drug, and/or a protein degrading IgG.

In a specific embodiment, a target of the antibody is selected from the group consisting of a cell surface protein, cytokine, hormone, enzyme, intracellular messenger, intercellular messenger, and immune checkpoint.

In a specific embodiment, the composition of the present invention further comprises a viral vector drug or a gene therapy drug, preferably the viral vector drug is selected from the group consisting of an oncolytic virus, gene therapy virus, and a viral vector vaccine.

In a specific embodiment, the composition of the present invention further comprises a drug capable of lowering the level of IgG in the blood.

The present invention also provides a product comprising the variant, protein, and/or composition of the present invention and an additional therapeutic agent; the additional therapeutic agent is selected from the group consisting of a viral vector drug, an antibody, a polypeptide drug capable of lowering the level of IgG in the blood.

The present invention also provides a kit or kit-of-parts comprising: 1) a therapeutically effective amount of a drug comprising the variant, protein, and/or composition of the present invention; and 2) a therapeutically effective amount of the additional therapeutic agent; the additional therapeutic agent is selected from the group consisting of a viral vector drug, an antibody, a polypeptide drug capable of lowering the level of IgG in the blood; the viral vector drug is preferably an oncolytic virus, a gene therapy virus.

The kit can comprise instructions relating to the administration (e.g. dosage information, dosing interval information) of a therapeutically effective amount of the variant, protein, and/or composition of the present invention and a therapeutically effective amount of the additional therapeutic agent. The additional therapeutic agent is selected from the group consisting of a viral vector drug, an antibody, and a polypeptide drug capable of lowering the level of IgG in the blood; the viral vector drug is preferably an oncolytic virus, a gene therapy virus.

### 5. Use of the Fc variant of the invention and method of treating disease thereof

The present invention also relates to a method of using the variant, protein, composition, and/or kit of the present invention for the treatment of diseases or use in the preparation of a drug. In some embodiments, the drug is used for treating an autoantibody-mediated disorder in a subject. In some embodiments, the drug is used for lowering the IgG level in a subject, and the IgG in blood is human blood immunoglobulin G and/or therapeutic immunoglobulin G. In some embodiments, the drug is used for preventing and/or treating autoantibody-mediated organ rejection following solid organ transplantation in a subject. In some embodiments, the drug is used for gene therapy. In some embodiments, the drug is used for eliminating neutralizing antibodies from pre-existing antiviral vectors in vivo prior to a viral vector-based gene therapy. In some embodiments, the drug is used for treating tumors in a subject. In some embodiments, the drug is used for clearing autoantibodies in a subject so that the Fc-containing agent is more therapeutically effective. In some embodiments, the drug is used for reducing the level of an Fc-containing agent in a subject's serum to whom the Fc-containing agent has been administered.

The present invention provides use of the Fc variant of the present invention, protein, composition, or kit containing the variant in the preparation of a drug for reducing the level of IgG in subjects or in its blood. The present invention also provides use of the Fc variant of the present invention or the protein, composition, or kit containing such variant in the preparation of a drug for treating autoimmune and/or inflammatory disease. The use includes administering a therapeutic effective amount of the Fc variant of the present invention or the encoded nucleic acid to a subject in need. The present invention also provides a method of using the Fc variant of the present invention, or the protein, composition, or kit comprising the variant for treating an antibody-mediated disorder, preferably an autoimmune and/or inflammatory disease.

In some embodiments, the autoimmune disease is selected from: Addison's disease, alopecia areata, ankylosing spondylitis, antiphospholipid syndrome, aplastic anemia, anti-GBM glomerulonephritis, anti-NMDAR encephalitis, antiphospholipidemic syndrome, autoimmune gastritis, autoimmune hearing loss, autoimmune hemolytic anemia, autoimmune hepatitis, autoimmune hypoparathyroidism, autoimmune hypophysitis, autoimmune inner ear disease, autoimmune lymphoproliferative syndrome, autoimmune myocarditis, autoimmune oophoritis, autoimmune bullous skin disease, autoimmune orchitis, autoimmune polyendocrinopathy, Behcet's disease, bullous pemphigoid, cardiomyopathy, inflammatory demyelinating polyneuropathy, acute motor axonal neuropathy, Churg-Strauss syndrome, abdominal diseases, autoimmune urticaria, Crohn's disease, CREST syndrome, celiac disease, Degos's disease, anti-neutrophil cytoplasmic antibody associated vascular inflammation, autoimmune neutropenia, acquired epidermolysis bullosa, essential mixed cryoglobulinemia, giant cell arteritis, glomerulonephritis, Goodpasture's syndrome, Graves' disease, Guillain-Barre syndrome, Hashimoto's thyroiditis, primary immunologic thrombocytopenic purpura, inflammatory bowel disease, Kawasaki disease, Ménière's syndrome, mixed connective tissue disease, Mooren ulcer, rheumatoid arthritis, multiple sclerosis, myasthenia gravis, stiffman syndrome, complete congenital heart block, acquired epidermolysis bullosa, pemphigus foliaceus, pemphigus vulgaris, pernicious anemia, polyarteritis nodosa, types of polyglandular autoimmune syndrome, primary biliary cirrhosis, types of polyglandular autoimmune syndrome, types of polyglandular autoimmune syndrome, polymyositis/dermatomyositis, psoriasis, psoriatic arthritis, Raynaud's syndrome, Reiter's syndrome, rheumatic heart disease, hemophilia-acquired FVIII deficiency, Lambert-Eaton myasthenia syndrome, multiple myeloma, sarcoidosis, collagen thesaurismosis, Sjogren's syndrome, subacute thyroiditis, sympathetic ophthalmia, systemic lupus erythematosus, Takayasu's arteritis, Sjögren's syndrome, type I diabetes, vitiligo, Vogt-Koyanagi-Harada syndrome or Wegener's granulomatosis, acute asthma or chronic asthma, organ transplant rejection, primary progressive multiple sclerosis, systemic sclerosis, serological disease, and immune complex hypersensitivity; wherein the immune complex is an immune complex formed by an anti-drug antibody and a drug. In some embodiments, the autoimmune disease is selected from: anti-glomerular basement membrane antibody disease (anti-GBM antibody disease), pathogenic IgG antibody-mediated thrombotic thrombocytopenia, Guillain-Barre syndrome (GBS), autoimmune encephalitis (AE), neuromyelitis optica lineage disease (NMOSD), atypical hemolytic uremic syndrome (aHUS), antibody-mediated rejection after organ transplantation (AMR), myasthenia gravis (MG); wherein the pathogenic IgG antibody mediated thrombotic thrombocytopenia is preferably thrombotic thrombocytopenic purpura (TTP), heparin induced thrombocytopenia (HIT), vaccine induced immune thrombotic thrombocytopenia (VITT).

In some embodiments, the antibody-mediated disorder is treatable using intravenous immunoglobulin (IVIG), subcutaneous immunoglobulin (SCIG), plasmapheresis, and/or immunoadsorption. In some embodiments, the antibody-mediated disorder is hyperglobulinemia. In some embodiments, the hyperglobulin is produced by a leukocyte selected from the group consisting of B cells and abnormal B cells; the globulin is selected from the group consisting of a gamma globulin; the hyperglobulinemia comprises primary monoclonal gammaglobulinemia, connective tissue disease, liver disease, infectious disease, sarcoidosis, myasthenia gravis, Hodgkin's disease, Behcet's disease, nephritis, allergic purpura, immune (or idiopathic) thrombocytopenia, malignant monoclonal gammaglobulinemia (such as multiple myeloma, heavy chain disease, malignant lymphoma, chronic lymphocytic leukemia, macroglobulinemia, etc.), secondary monoclonal gammaglobulinemia (such as non-lymphoreticular tumor, monocytic leukemia, cryoglobulinemia, etc.), benign M-proteinemia, monoclonal gammopathy of unknown significance (MGUS), Waldenstrom's macroglobulinemia, AL-type amyloidosis, solitary plasmacytoma (bone or extraosseous), POMES syndrome, reactive plasmacytosis, osteolytic lesions of metastatic cancer, plasmoblastic lymphoma, monoclonal immunoglobulin-associated renal damage (MGRS), etc. The condition is preferably multiple myeloma.

Host IgG causes acute transplant rejection in organ transplantation. Transplant rejection is divided into two cases, one is host versus graft reaction (HVGR) and the other is graft versus host reaction (GVHR). In solid organ transplants, host versus graft reactions are predominant and graft versus host reactions are rare. In bone marrow transplantation, graft-versus-host reactions are common. Graft-versus-host reaction (GVHR) refers to the recognition by the recipient's immune system of a graft, such as a foreign tissue or organ, as a heterologous component after the recipient has undergone an allograft or organ transplant, which initiates an immunological response to the attack, destruction and clearance of the graft. For patients with high sensitivity to human lymphocyte antigen (HLA), transplant rejection is more likely to occur. The mechanism of rejection mainly includes two aspects: cellular immunity and humoral immunity, in which humoral immunity is the immune mechanism of IgG produced by effector B cells for the purpose of protection.

The present invention also provides use of the Fc variant of the present invention, or the protein, composition, or kit comprising the variant in the preparation of a drug for preventing and/or treating autoantibody-mediated organ rejection after solid organ transplantation in a subject. In some embodiments, the organ is selected from the group consisting of the kidney, pancreatic islet, pancreas, heart, liver, lung, or small intestine.

In viral vector-based gene therapy, some patients already have anti-viral antibodies in the body before treatment, and the neutralizing antibodies may lead to the decrease or even failure of gene therapy. On the other hand, patients who have received gene therapy using viruses as carriers may develop antiviral antibodies, leading to the inability to administer medication in the short term. The variant of the present invention solves this problem. The present invention provides use of the Fc variant of the present invention, the protein, the composition or kit comprising the Fc variant of the present invention in the preparation of a drug for the clearance of a neutralizing antibody of a pre-existing antiviral vector in vivo prior to viral vector-based gene therapy.

### Administration of pharmaceutical composition and therapeutic method

The present invention also relates to a method of administering the variant, protein, pharmaceutical composition, and/or kit of the present invention and method of administering the variant, protein, pharmaceutical composition, and/or kit of the present invention to treat a disease or disorder.

Preferably, the variant, protein, and/or composition of the present invention may be administered prior to the administration of an additional therapeutic agent in the body that has developed anti-drug antibodies or is susceptible to developing anti-drug antibodies. Preferably, the variant, protein, and/or composition of the present invention may be administered prior to, simultaneously with, and/or after administration of an anti-drug antibody or an additional therapeutic agent susceptible to anti-drug antibody production in the body.

In the use of the present invention, the protein and/or composition of the present invention and the additional therapeutic agent are present as a combined preparation for simultaneous, separate or sequential use.

In some embodiments, the method comprises the steps of: 1) administering the variant, protein, and/or composition of the present invention to a subject; subsequently, 2) administering the additional therapeutic agent to the subject. Preferably, the variant, protein, and/or composition of the present invention is separated in time from the administration of the additional therapeutic agent.

In some embodiments, the method comprises the steps of 1) administering an additional therapeutic agent to a subject; subsequently, 2) administering the variant, protein, and/or composition of the present invention to the subject. Preferably, the variant, protein, and/or composition of the present invention is separated in time from the administration of the additional therapeutic agent. Preferably, the variant, protein, and/or composition of the present invention are administered in amounts and at intervals sufficient to reduce immunoglobulin levels in the subject to 60% of the starting level. More preferably, the variant, protein, and/or composition of the present invention are administered in amounts and at intervals sufficient to reduce immunoglobulin level binding in the subject to less than 50%, 40%, 30%, 20%, or 10% of the starting level in the patient. The variant, protein, and/or composition of the present invention may be administered at a single point in time or within a set period of time.

Preferably, wherein the variant, protein, and/or composition of the present invention are administered by intravenous infusion, intraperitoneal injection, intramuscular injection, articular injection, intradermal injection, or subcutaneous injection. The preferred mode of injection is intravenous infusion. And/or the amount of variant, protein, and/or composition of the present invention administered is from 1 mg/kg body weight to 100 mg/kg body weight, from 4 to 50mg/kg body weight, from 5 mg/kg body weight to 50 mg/kg body weight, or from 5 mg/kg body weight to 10 mg/kg body weight.

Preferably, the variant, protein, and/or composition of the present invention and the additional therapeutic agent are administered at a time interval of at least 30 minutes, at least 1 hour, at least 2 hours, at least 3 hours, at least 4 hours, at least 4 hours, at least 5 hours or at least 6 hours; and up to 35 days, up to 28 days, up to 21 days, up to 18 days, up to 14 days, up to 13 days, up to 12 days, up to 11 days, up to 10 days, up to 9 days, up to 8 days, up to 7 days, up to 6 days, up to 5 days, up to 4 days, up to 3 days, up to 2 days, up to 24 hours, up to 18 hours, up to 12 hours, up to 10 hours, up to 8 hours, up to 7 hours, or up to 6 hours.

In another embodiment, the method comprises the steps of 1) ex vivo treating the blood from the subject with the variant, protein, and/or composition of the present invention; 2) returning blood to the subject; and 3) administering the additional therapeutic agent to the subject.

In another embodiment, the method comprises the steps of: 1) administering the additional therapeutic agent to the subject; 2) ex vivo treating the blood from the subject with the variant, protein, and/or composition of the present invention; and 3) returning the blood to the subject.

In a preferred embodiment, the variant, protein, and/or composition of the present invention are used in combination with an additional therapeutic agent for the prevention and/or treatment of cancer.

In a preferred embodiment, the variant, protein, and/or composition of the present invention are used in combination with an additional therapeutic agent for the prevention and/or treatment of a genetic defect related disease.

In a preferred embodiment, the variant, protein, and/or composition of the present invention are used in combination with an additional therapeutic agent for the treatment of a disease or condition mediated by a pathogenic IgG antibody.

The present invention also provides a method of administering the pharmaceutical combination to a subject to treat or prevent a gene deficiency related disease, cancer, infection, or a disease or condition mediated by a pathogenic IgG antibody. The method results in a reduction of 20-50%, 50-75%, 75-90%, 90-95%, or 95% or more of the antibody bound by the viral vector; the method results in a 20-50%, 50-75%, 75-90%, 90-95% or 95% or more reduction in the pathogenic IgG antibody. Preferably, the pharmaceutical combination is for use in a method of treating a disease associated with a genetic deficiency. Preferably, the pharmaceutical combination is for use in a method of treating a gene deficiency related disease. The preferred infections are viral, bacterial, or fungal infections. Preferably, the pharmaceutical combination is for use in a method of treating cancer. Preferably, the drug is for use in a method of treating a disease or condition mediated by a pathogenic IgG antibody.

Preferably, the other therapeutic agent is a viral vector drug; Preferably, the viral vector drug can be an oncolytic virus, viral vaccine, and gene therapy virus.

Preferably, the additional therapeutic agent is an antibody.

Preferably, the additional therapeutic agent is a polypeptide drug that can reduce the level of IgG in the blood, such as an immunoglobulin degrading enzyme. More preferably, the polypeptide drug that can reduce the level of IgG in the blood is used in the treatment of the above-mentioned diseases mediated by a pathogenic IgG antibody.

Preferably, the components of the pharmaceutical combination are administered separately or the components of the pharmaceutical combination are administered simultaneously.

In these uses and methods as described above, further combinations with additional therapeutic agents, such as an anti-inflammatory agent, are also possible.

In some embodiments, the additional therapeutic agent is a leukocyte depleting agent.

In some embodiments, the additional therapeutic agent is a B-cell depleting agent.

In some embodiments, the B-cell depleting agent is an antibody, preferably an antibody for tumor therapy, more preferably the antibody specifically binds to CD10, CD19, CD20, CD21, CD22, CD23, CD24, CD37, CD53, CD70, CD72, CD74, CD75, CD77, CD79a, CD79b, CD80, CD81, CD82, CD83, CD84, CD3, CD11a, CD14, CD25, CD28, CD30, CD33, CD36, CD38, CD40, CD44, CD52, CD55, CD59, CD56, CD103, CD134, CD137, CD138, CD152, CD3, IL-1p, IL-2R, IL-6, IL-6R, IL-12, IL-23, C5, BAFF, BLyS, BCMA, CXCR-4, ICAM-1, SLAMF7/CS1, TNFα, IgE, CD85, or CD86.

In some embodiments, the additional therapeutic agent is rituximab, daclizumab, basiliximab, muromonab-CD3, infliximab, adalimumab, omalizumab, efalizumab, natalizumab, tocilizumab, eculizumab, golimumab, canakinumab, Ustekinumab, belimumab, daratumumab, Isatuximab, or a combination thereof.

In some embodiments, the Fc-containing agent is a therapeutic or diagnostic agent, preferably the Fc-containing agent is an antibody, Fc fusion protein antibody-drug conjugate.

### 6. Advantages and beneficial effects of the present invention

The Fc variant of the present invention has the following advantages over the control variants: 1) has an increased anti-inflammatory property; 2) has an unexpectedly increased level of sialic acid modification without affecting FcRn affinity; 3) can reduce the level of IgG in the blood; 4) has an increased area under the concentration-time curve; 5) has constant or increased binding for FcyRI; and/or, 6) has increased selectivity of binding for FcyRI in various Fcy receptors.

### Examples

On the basis of complying with common knowledge in the field, the various conditions described above can be arbitrarily combined to obtain various preferred examples of the present invention.

The present invention is further illustrated by way of the following examples, without thereby limiting the present invention to the scope of the described examples. The experimental methods in the following embodiments that do not specify specific conditions shall be selected according to conventional methods and conditions, or according to the product manual. The reagents and raw materials used in the present invention are commercially available.

### Example 1: Protein preparation

Sequence synthesis: the Fc variants with different mutations in Table 1 were synthesized and constructed into a mammalian cell expression vector pcDNA3.1. To achieve the highest 2,6-sialic acid modification in CHO cells, synthetic human galactosyltransferase (β1,4-galactosyltransferase, abbreviated GT) and human α2,6-sialyltransferase (ST6Gal1, abbreviated ST) enzymes were expressed along with the variant.

Expression and purification: the different variant combinations of GT and ST were transiently transfected into ExpiCHO-S cells at the same time, and supernatants were collected from the 6th to 8th day of transfection by centrifugation at 9500 rpm for 20 minutes. Purification was done with ProteinA.

**Table 1: Fc variants with different mutations**

| SEQ ID NO: | Variant abbreviation | Amino Acid Sequence |
|---|---|---|
| SEQ ID NO: 1 | m1 | DKTHTCPPCPAPELLGGPSVFLFPPKPKDTLYITREPEVTCVVVDVSH EDPEVKFNWYVDGVEVHNAKTKPREEQYNSTYRVVSVLTVLHQDW LNGKEYKCKVSNKALPAPIEKTISKAKGQPREPQVYTLPPSRDELTKN QVSLTCLVKGFYPSDIAVEWESNGQPENNYKTTPPVLDSDGSFFLYSK LTVDKSRWQQGNVFSCSVMHEALKFHYTQKSLSLSP |
| SEQ ID NO: 2 | m2 | DKTHTCPPCPAPELLGGPSVALFPPKPKDTLMISRTPEVTCVVVDVSH EDPEVKFNWYVDGVEVHNAKTKPREEQYNSTYRVVSVLTVLHQDW LNGKEYKCKVSNKALPAPIEKTISKAKGQPREPQVYTLPPSRDELTKN QVSLTCLVKGFYPSDIAVEWESNGQPENNYKTTPPVLDSDGSFFLYSK LTVDKSRWQQGNVFSCSVMHEALHNHYTQKSLSLSP |
| SEQ ID NO: 3 | m3 | DKTHTCPPCPAPELLGGPSVALFPPKPKDTLYITREPEVTCVVVDVSH EDPEVKFNWYVDGVEVHNAKTKPREEQYNSTYRVVSVLTVLHQDW LNGKEYKCKVSNKALPAPIEKTISKAKGQPREPQVYTLPPSRDELTKN QVSLTCLVKGFYPSDIAVEWESNGQPENNYKTTPPVLDSDGSFFLYSK LTVDKSRWQQGNVFSCSVMHEALKFHYTQKSLSLSP |
| SEQ ID NO: 4 | m4 | DKTHTCPPCPAPELLGGPSVFLAPPKPKDTLYITREPEVTCVVVDVSH EDPEVKFNWYVDGVEVHNAKTKPREEQYNSTYRVVSVLTVLHQDW LNGKEYKCKVSNKALPAPIEKTISKAKGQPREPQVYTLPPSRDELTKN QVSLTCLVKGFYPSDIAVEWESNGQPENNYKTTPPVLDSDGSFFLYSK LTVDKSRWQQGNVFSCSVMHEALKFHYTQKSLSLSP |
| SEQ ID NO: 5 | m5 | DKTHTCPPCPAPELLGGPSVFLFPPKPKDTLYITREPEVTCVVVDVSH EDPEVKFNWYVDGVEVHNAKTKPREEQYNSTYRVVSVLTVLHQDWL NGKEYKCKVSNKALPAPIEKTISKAKGQPREPQVYTLPPSRDELTKN QVSLTCLVKGFYPSDIAVEWESNGQPENNYKTTPPVLDSDGSFFLYSK LTVDKSRWQQGNVFSCSVMHEALKFHYTQKSLSLSP |
| SEQ ID NO: 6 | m6 | DKTHTCPPCPAPELLGGPSVFLFPPKPKDTLYITREPEVTCVVADVSH EDPEVKFNWYVDGVEVHNAKTKPREEQYNSTYRVVSVLTVLHQDW LNGKEYKCKVSNKALPAPIEKTISKAKGQPREPQVYTLPPSRDELTKN QVSLTCLVKGFYPSDIAVEWESNGQPENNYKTTPPVLDSDGSFFLYSK LTVDKSRWQQGNVFSCSVMHEALKFHYTQKSLSLSP |
| SEQ ID NO: 7 | m7 | DKTHTCPPCPAPELLGGPSVFLFPPKPKDTLYITREPEVTCVVEDVSH EDPEVKFNWYVDGVEVHNAKTKPREEQYNSTYRVVSVLTVLHQDW LNGKEYKCKVSNKALPAPIEKTISKAKGQPREPQVYTLPPSRDELTKN QVSLTCLVKGFYPSDIAVEWESNGQPENNYKTTPPVLDSDGSFFLYSK LTVDKSRWQQGNVFSCSVMHEALKFHYTQKSLSLSP |
| SEQ ID NO: 8 | m8 | DKTHTCPPCPAPELLGGPSVFLFPPKPKDTLYITREPEVTCVVVDVSH EDPEVKFNWYVDGVEVHNAKTKPREEQANSTYRVVSVLTVLHQDW LNGKEYKCKVSNKALPAPIEKTISKAKGQPREPQVYTLPPSRDELTKN QVSLTCLVKGFYPSDIAVEWESNGQPENNYKTTPPVLDSDGSFFLYSK LTVDKSRWQQGNVFSCSVMHEALKFHYTQKSLSLSP |
| SEQ ID NO: 9 | m9 | DKTHTCPPCPAPELLGGPSVFLFPPKPKDTLYITREPEVTCVVVDVSH EDPEVKFNWYVDGVEVHNAKTKPREEQNASTYRVVSVLTVLHQDW LNGKEYKCKVSNKALPAPIEKTISKAKGQPREPQVYTLPPSRDELTKN QVSLTCLVKGFYPSDIAVEWESNGQPENNYKTTPPVLDSDGSFFLYSK LTVDKSRWQQGNVFSCSVMHEALKFHYTQKSLSLSP |
| SEQ ID NO: 10 | m10 | DKTHTCPPCPAPELLGGPSVFLFPPKPKDTLYITREPEVTCVVVDVSH EDPEVKFNWYVDGVEVHNAKTKPREEQYNNASRVVSVLTVLHQDW LNGKEYKCKVSNKALPAPIEKTISKAKGQPREPQVYTLPPSRDELTKN QVSLTCLVKGFYPSDIAVEWESNGQPENNYKTTPPVLDSDGSFFLYSK LTVDKSRWQQGNVFSCSVMHEALKFHYTQKSLSLSP |
| SEQ ID NO: 11 | m11 | DKTHTCPPCPAPELLGGPSVFLFPPKPKDTLYITREPEVTCVVVDVSH EDPEVKFNWYVDGVEVHNAKTKPREEQYNSTARVVSVLTVLHQDW LNGKEYKCKVSNKALPAPIEKTISKAKGQPREPQVYTLPPSRDELTKN |
| | | QVSLTCLVKGFYPSDIAVEWESNGQPENNYKTTPPVLDSDGSFFLYSK LTVDKSRWQQGNVFSCSVMHEALKFHYTQKSLSLSP |
| SEQ ID NO: 12 | Fc(G1m3) | DKTHTCPPCPAPELLGGPSVFLFPPKPKDTLMISRTPEVTCVVVDVSH EDPEVKFNWYVDGVEVHNAKTKPREEQYNSTYRVVSVLTVLHQDW LNGKEYKCKVSNKALPAPIEKTISKAKGQPREPQVYTLPPSREEMTK NQVSLTCLVKGFYPSDIAVEWESNGQPENNYKTTPPVLDSDGSFFLYS KLTVDKSRWQQGNVFSCSVMHEALHNHYTQKSLSLSP |
| SEQ ID NO: 13 | Fc(G1m1, 17) | DKTHTCPPCPAPELLGGPSVFLFPPKPKDTLMISRTPEVTCVVVDVSH EDPEVKFNWYVDGVEVHNAKTKPREEQYNSTYRVVSVLTVLHQDW LNGKEYKCKVSNKALPAPIEKTISKAKGQPREPQVYTLPPSRDELTKN QVSLTCLVKGFYPSDIAVEWESNGQPENNYKTTPPVLDSDGSFFLYSK LTVDKSRWQQGNVFSCSVMHEALHNHYTQKSLSLSP |

### Example 2. Variant sialic acid modification detection

Sambucus Nigra Lectin (SNA) lectin binds specifically to sialic acid, with 2,6 linked sialic acid binding better than 2,3 linked sialic acid. The binding of the variants to SNA was detected by means of ELISA. 0.5 µg of the purified variant protein was coated onto an enzyme-linked immunosorbent assay plate and incubated with 300 µl of 1% PVA (PBS) solution for 2 hours at 37°C. SNA-Biotin with PBST (Biotinylated Sambucus Nigra Lectin, Vectorlab, Cat. No. B-1305-2) was diluted to 2 µg/ml, 100 µl per well was allowed to bind for 1 hour at 37°C.

HRP was diluted with PBST to mark Streptavidin (Beyotime, Cat. No. A0303) 1000 folds. 100 µl per well was allowed to bind for 1 hour at 37°C, followed by the colour development.

Results showed that different combinations of mutations had different effects on the sialic acid modifications. Among them, sialic acid increased the positive control m2, and compared to m1 without related mutations, the increase in sialic acid modification was limited. It has also been unexpectedly found that the combination variants m3-m7 and m11, had significantly increased sialic acid modifications compared to the positive control m2.

**Table 2: increased 2,6 linked sialic acid in different Fc variants**

| Variants | 2,6-SA increase (OD450 nm) |
|---|---|
| m1 | 0.62 |
| m2 | 1.08 |
| m3 | 2.44 |
| m4 | 2.30 |
| m5 | 2.34 |
| m6 | 2.10 |
| m7 | 2.34 |
| m8 | 0.04 |
| m9 | 0.05 |
| m10 | 0.08 |
| m11 | 1.71 |

### Example 3. Binding of variants and FcRn

The variant proteins were tested for affinity to FcRn at pH 7.0. The affinity of the antibody fragment for the receptor was determined using Fortebio. FcRn (Aero Biosystems) was loaded at 0.8 nm with an HIS1K probe, bound to the variant protein at various concentrations for 60 seconds and dissociated for 300 seconds.

The results showed that variants m2 and m11 had a lower neutral affinity for FcRn. The affinity level between other variants and FcRn is similar to that of the control m1, and the Kd value is similar. This suggests that an increased mutation in m11 as compared to control m1 may negatively affect Fc binding to FcRn.

**Table 3: Specific binding of different Fc variants to FcRn**

| Variants | FcRn Binding Ka (1/Ms) |
|---|---|
| m1 | 2.14E+05 |
| m2 | 1.18E+04 |
| m3 | 2.72E+05 |
| m4 | 2.98E+05 |
| m5 | 3.47E+05 |
| m6 | 2.12E+05 |
| m7 | 2.21E+05 |
| m8 | 1.15E+05 |
| m9 | 2.08E+05 |
| m10 | 2.21E+05 |
| m11 | 3.83E+04 |

### Example 4. Variant stability assay

The high-throughput protein stability assay system Uncle was used to detect the thermostability of the variant. 1.0 mg/ml of purified variant sample was loaded for online detection: the Tm & Tagg with optional DLS program was used, the temperature interval was set at 25-95°C, and a duplicate well was set for the sample. Thermal stability was analyzed by analyzing the resulting parameters Tm (protein melting temperature) and Tagg266 (protein aggregation temperature).

Results: except for m4, the other variants all had Tm1 values above 50°C. These results suggest that different combinations of mutations can have different effects on the thermostability of proteins, and some mutations can decrease the thermostability of Fc variants.

**Table 4. Thermal stability of different Fc variants**

| Variants | Concentration (mg/ml) | Tonset (°C) | Tm1 (°C) | Tm2 (°C) | Tm3 (°C) | Tagg 266 (°C) |
|---|---|---|---|---|---|---|
| m1 | 0.74 | 49.28 | 61.00 | 61.00 | 78.40 | 77.06 |
| m2 | 0.99 | 57.43 | 61.35 | 69.40 | 80.00 | 65.10 |
| m3 | 0.63 | 46.36 | 50.94 | 69.00 | 78.37 | 70.90 |
| m4 | 0.69 | 43.15 | 48.20 | 68.10 | 78.71 | 66.08 |
| m5 | 0.69 | 47.47 | 58.60 | 67.60 | 78.78 | 70.04 |
| m6 | 0.71 | 41.25 | 55.11 | 67.33 | 78.57 | 69.30 |
| m7 | 0.65 | 46.88 | 55.45 | 63.10 | 77.00 | 74.70 |
| m8 | 0.22 | 54.74 | 61.89 | 61.89 | 76.99 | 76.50 |
| m9 | 0.16 | 59.71 | 65.65 | 65.65 | 78.39 | -- |
| m10 | 0.27 | 50.13 | 55.40 | 55.40 | 78.26 | 80.33 |
| m11 | 0.12 | 48.62 | 52.91 | 68.60 | 78.70 | -- |

### Example 5. Binding of variants and FcγR

The variant proteins were tested for affinity to FcyRI, FcyRIIa-131H, FcyRIIa-131R, FcyRIIb, FcyRIIIa-158F, FcyRIIIa-158V at pH 7.0. The affinity of the antibody fragment for the receptor was determined using Fortebio. Different FcyR (Aero Biosystems) was loaded at 0.5 nm with an HISlK probe, bound to the variant protein at various concentrations for 60 seconds and dissociated for 60 seconds.

**Table 5: Affinity of different variants for FcyR receptors**

| FcγR Name | Fc (G1m3) KD (nM) | m1 KD (nM) | m6 KD (nM) | Fold change of m6 relative to m1 | m7 KD (nM) | Fold change of m7 relative to m1 |
|---|---|---|---|---|---|---|
| FcγRI | 4.07E-08 | 3.16E-07 | 3.19E-07 | **0.99** | 6.38E-08 | 4.95 |
| FcγRIIa-131H | 2.58E-06 | 2.56E-06 | 2.56E-06 | **1.00** | * | - |
| FcγRIIa-131R | 1.72E-06 | 2.05E-06 | 1.40E-06 | **1.46** | - | - |
| FcγRIIIa-158V | 1.49E-06 | 1.71E-06 | 1.76E-06 | **0.97** | - | - |
| FcγRIIIa-158F | 4.68E-06 | 7.21E-06 | 9.67E-06 | **0.75** | - | - |
| FcγRIIb | 2.11E-06 | 1.07E-05 | 6.82E-06 | **1.57** | - | - |

| | | | | | | |
|---|---|---|---|---|---|---|
| * Note: no binding, or binding below quantitation limit. | | | | | | |

The results showed that the m6 variants all had less than a 2-fold change in binding to FcyR relative to the control m1. Whereas m7 had a 5-fold increase in binding to FcyRI, and there was a significant increase in selectivity for binding to FcyRI in a variety of Fcγ receptors.

### Example 6. Variant sialic acid content detection

The acid hydrolysis method was used to release the bound sialic acid in glycoproteins into a free state. The free-state sialic acid was bound with the fluorescent dye DMB (Sigma, Cat. No. D4784-50 mg) to obtain derivatives with fluorescence characteristics. High performance liquid chromatography (HPLC) was used to detect them under an excitation wavelength of 373 nm and an absorption wavelength of 448 nm. The sialic acid standard (TCI, Cat. No. A1105) was used in 5 concentrations: 100 pmol/µl, 80 pmol/µl, 60 pmol/µl, 40 pmol/µl, and 20 pmol/µl.

The results showed that the sialic acid content of m4, m5, m6, and m7 was significantly higher than that of m1.

**Table 6: sialic acid contents of different variants**

| Variants | Sialic acid content (mol/mol protein) | Sialic acid content (mol/mol N-glycan) |
|---|---|---|
| m1 | 0.84 | 0.42 |
| m4 | 3.20 | 1.60 |
| m5 | 2.52 | 1.26 |
| m6 | 3.00 | 1.50 |
| m7 | 2.91 | 1.45 |

The N-glycan types of different variant proteins were detected by 2-AB labeling-chromatography after digestion. Protein samples were subjected to neuraminidase (Rhino Biotechnology, Cat. No. QPF-005) digestion as an asialo-modified sample control to confirm the sialic acid N glycoform peak.

The different variant proteins were diluted to 2 mg/ml and exchanged with 50 mM NaH₂PO₄ buffer, pH = 7.5. The N-glycoform of the protein was digested with PNGaseF (Rhino Biotechnology, Cat. No. QPF-001) and incubated at 37°C for 24 h. The digested sample was labeled with 2-AB to obtain a sample for HPLC detection. Detection was performed using a WATERS Acquity UPLC BEH Amide analytical column with wavelength λex = 330 nm and λem = 420 nm. Experiments were performed using 100 mM ammonium formate, (pH 4.5), 100% acetonitrile as the mobile phase.

The results showed that the modification proportion of sialic acid in m4, m5, m6, and m7 was significantly higher than that in m1.

**Table 7: sialic acid modification proportion of different variants**

| Variants | G0F | G1F | G1F' | G2F | Peak 1 | Peak 2 | Peak 3 | Peak 4 | SA modification proportion (%) | Percentage of SA after neuraminidase treatment (%) |
|---|---|---|---|---|---|---|---|---|---|---|
| m1 | 59.7 | 17.9 | 5.0 | 1.0 | 1.7 | 7.4 | 0.05 | 1.5 | 10.7 | 1.3 |
| m4 | 1.1 | 5.5 | 0.1 | 9.1 | 10.9 | 1.1 | 26.5 | 39.0 | 77.5 | 1.0 |
| m5 | 1.3 | 9.9 | 0.2 | 5.4 | 12.6 | 1.5 | 33.0 | 29.7 | 76.7 | 0.8 |
| m6 | 1.4 | 5.4 | 0.1 | 3.4 | 16.5 | 1.4 | 19.4 | 45.1 | 82.4 | 0.7 |
| m7 | 3.3 | 14.3 | 0.6 | 15.6 | 8.7 | 1.8 | 37.0 | 12.5 | 60.0 | 1.3 |

### Example 7. In vivo administration of variant proteins

The therapeutic effects of the variants were observed in a mouse model of acute immune thrombocytopenia. C57BL/6 mice were injected once with 0.1 µg/g of the antiplatelet antibody 6A6-IgG1. 2h after injection with 1 g/kg of IVIG or 0.1 g/kg of the variants (m1, m4, m5, m6), and PBS as a control. The platelet content of mice was detected before administration of 6A6-IgG1 antibody and at 4h after administration, and the ratio of platelet content at 4 h to platelet content before administration was used as an evaluation index.

The results showed that only IVIG (72 ± 15%), m4 (69 ± 18%), m5 (77 ± 20%), and m6 (65 ± 25%) showed platelet recovery effect, while m1 (19 ± 22%) had no significant difference compared with the PBS control group (25 ± 15%). It demonstrated that the m4-m7 variants had significant anti-inflammatory effects in mice.

### Example 8. Administration of variant proteins in an experimental autoimmune encephalomyelitis model

C57BL/6 mice were immunized for induction of experimental autoimmune encephalomyelitis (EAE) using the following protocol: 2 mg/ml MOG35-55 and Complete Freund's Adjuvant (CFA, by adding 4 mg/ml Mycobacterium tuberculosis strain H37RA to Incomplete Freund's Adjuvant (IFA, sigma)) were mixed at a 1: 1 ratio in PBS to give an MOG35-55/CFA emulsifier. Each mouse was injected subcutaneously with 100 µL of emulsifier at the following sites: 40 µL on each side of the groin, 10 µL on each back instep, and then 200 ng/animal PTX were injected via the tail vein on day 2. Mice were dosed (i. p.) with different high sialic acid-modified variant proteins on days 5, 10, 15, and 20 post-immunization. Animals were followed for clinical signs of disease using the following scoring system:
Point 0: health in mice
Point 1: tail weakness in mice
Point 2: impaired righting reflex in mice
Point 3: one hind limb paralysis in mice
Point 4: two hind limbs paralysis in mice
Point 5: 20% and above of weight loss in mice
Point 6: death or moribund in mice (if moribund mice were found, they will be humanely sacrificed immediately)

**Table 8: EAE model experimental design**

| **Grouping** | **Number of animals** | **Drug** | **Dose (mg/kg)** | **Administratio n mode** | **Administration date** |
|---|---|---|---|---|---|
| 1 | 10 | PBS | / | i.p. | D5, 10, 15, 20 |
| 2 | 10 | m1 | 100 | i.p. | D5, 10, 15, 20 |
| 3 | 10 | m4 | 100 | i.p. | D5, 10, 15, 20 |
| 4 | 10 | m6 | 100 | i.p. | D5, 10, 15, 20 |
| 5 | 10 | IVIG | 1000 | i.p. | D5, 10, 15, 20 |

The experiment was terminated 28 days after the first immunization. The proportion of Treg cells in the spleen was analyzed by flow cytometry.

ELISA was used to detect the endpoint of the content of IgG in the blood. 0.5 µg of Rabbit anti-Mouse IgG Fc (Thermo, Cat. No. 31555) Protein was coated onto the ELISA plate and incubated with 300µl of 1% PVA (PBS) solution for 2 hours at 37°C. The serum samples were incubated after 500-fold dilution, thereafter Rabbit anti-Mouse F(ab')2-HRP (Thermo, Cat. No. 31451) was diluted to 0.2 µg/ml with PBST. 100 µl was added per well, bound at 37°C for 1 hour, and colour developed.

The end-point MOG-specific IgG content was detected by ELISA. 2 µg of the MOG polypeptide was coated onto the plate and incubated with 300 µl of 1% PVA (PBS) solution at 37°C for 2 hours. The serum samples were incubated after 100-fold dilution, thereafter Rabbit anti-Mouse F(ab')2-HRP (Thermo, Cat. No. 31451) was diluted to 0.4 µg/ml with PBS-T. 100 µl was added per well, bound at 37°C for 1 hour, and developed.

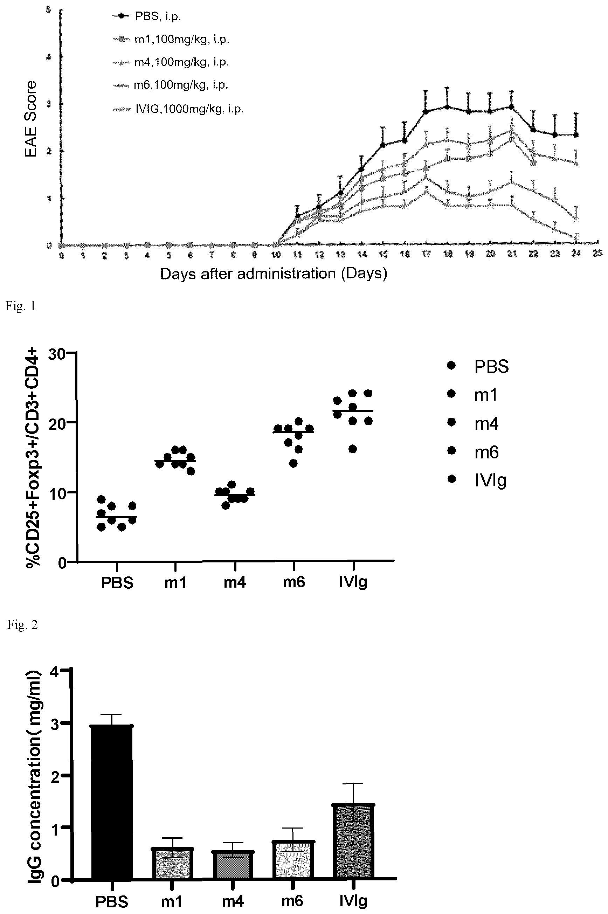

The results show that variants m4 and m6 of the present invention can decrease the disease score in the EAE mouse model (as shown in FIG. 1) and both can up-regulate the Treg cell proportion in the EAE mouse model (as shown in FIG. 2). Through the detection of the total IgG content in blood and the detection of the anti-MOG polypeptide IgG content, it was found that the two IgG contents were significantly down-regulated relative to the PBS group. It can be seen that the variants of the present invention are capable of achieving an anti-inflammatory effect comparable to that of IVIG.

The applicant declares that the present invention illustrates the detailed methods of the present invention through the above embodiments, but the present invention is not limited to the above detailed methods, which does not mean that the present invention must rely on the above detailed methods to be implemented. It will be apparent to those skilled in the art that any modifications to the present invention, equivalent alterations to the various raw materials of the products of the present invention, and additions of auxiliary components, selections of specific ways, etc. fall within the scope and disclosure of the present invention.

## Claims

1. A Fc variant comprising a 2,6-sialylated glycosylation modification, the variant having an increased affinity for FcRn compared to a wild-type IgG.

2. The Fc variant of claim 1, wherein the variant has an increased affinity for FcRn under neutral and/or weakly acidic conditions, preferably pH 6.5-7.4 for neutral conditions, preferably pH 5.0-6.5 for weakly acidic conditions.

3. The Fc variant of claim 1 or 2, wherein the Fc variant is obtained by mutation based on a wild-type IgG selected from the group consisting of IgG1, IgG2, IgG3, and IgG4, preferably the Fc variant is obtained by mutation based on the wild type IgG1 or an allotype thereof.

4. The Fc variant of any one of claims 1-3, wherein compared to a wild type Fc fragment of a wild type IgG1, the Fc variant comprises a mutation (EU numbering) selected from the group consisting of M252Y/S254T/T256E/H433K/N434F, M252Y/S254T/T256E, N434A, N434W, N434Y, M428L/N434S, T250Q/M428L, T307Q/N434A, M252Y/V308P/N434Y, T307P/L309Q/Q311R, H285D/T307Q/A378V, L309D/Q311H/N434S, M252Y/T256D, and T256D/T307Q, T256D/T307W.

5. The Fc variant of any one of claims 1-4, wherein compared to a wild type Fc fragment of a wild type IgG1, the Fc variant comprises a mutation (EU numbering) selected from the group consisting of V264A, Δ446G, Δ447K, V262E, ΔE293, ΔE294, V264E, F241A, F243A, and Y300A.

6. The Fc variant of any one of claims 1-5, wherein the Fc variant has an N-glycan attached at its glycosylation site, wherein the N-glycan is highly sialylated, preferably the glycosylation site is N297 (EU numbering).

7. The Fc variant of claim 6, wherein the N-glycan has a degree of 2,6-sialylation higher than 20%, preferably higher than 25%, preferably higher than 30%, preferably higher than 35%, preferably higher than 40%, preferably higher than 45%, preferably higher than 50%, preferably higher than 55%, preferably higher than 60%, preferably higher than 65%, preferably higher than 70%, preferably higher than 80%, preferably higher than 85%, preferably higher than 90%, more preferably higher than 95%.

8. The Fc variant of claim 6, wherein per mol Fc variant has a degree of 2,6-sialylation higher than 0.1 mol, higher than 0.2 mol, preferably higher than 0.3 mol, preferably higher than 0.4 mol, preferably higher than 0.5 mol, preferably higher than 0.6 mol, preferably higher than 0.7 mol, preferably higher than 0.8 mol, preferably higher than 0.9 mol, preferably higher than 1.0 mol, preferably higher than 1.1 mol, preferably higher than 1.2 mol, preferably higher than 1.3 mol per mole, preferably higher than 1.4 mol, preferably higher than 1.5 mol, preferably higher than 1.6 mol, preferably higher than 1.7 mol, preferably higher than 1.8 mol, preferably higher than 1.9 mol, more preferably higher than 2.0 mol.

9. The Fc variant of claim 6, wherein more than 40% of the N-glycan comprises one to four 2,6-sialic acid modifications at the end of the sugar chain, and less than 60% glycosyls of the N-glycan are in the form of G0, G1, G0F, G1F, G2, or G2F, where G0-G2 separately represents glycosyls that have been glycosylated by 0-2 galactoses, and F represents glycosyls that have been glycosylated by fucose.

10. The Fc variant of any one of claims 1-9, wherein the 2,6-sialic acid is N-acetylneuraminic acid (NANA), N-glycolylneuraminic acid (NGNA) or a mixture thereof, preferably wherein the 2,6-sialic acid is NANA.

11. The Fc variant of claim 10, wherein the Fc variant does not comprise an alpha-galactose modification.

12. The Fc variant of any one of claims 1-11, wherein the Fc variant has a fucose content of less than 50%, preferably less than 10%.

13. A protein comprising the Fc variant of any one of claims 1-12 and an optional element beneficial to the expression of the Fc variant; preferably, the protein contains a signal peptide at the N-terminus of the Fc variant and/or a histidine tag at the C-terminus of the Fc variant; more preferably, the protein is connected to a secretion signal sequence at the N-terminus of the Fc variant and, a methionine is connected to the N-terminus of the secretion signal sequence; more preferably, the protein comprises or consists of, from the N-terminus to the C-terminus: methionine, secretion signal sequence, and the Fc variant.

14. A composition or kit comprising the Fc variant of any one of claims 1-12 or the protein of claim 13.

15. A method for preparing the Fc variant of any one of claims 1-12, comprising the following steps:
(a) mutating a wild-type Fc fragment of a wild-type IgG1 to obtain a Fc variant, wherein the sequence of the wild-type Fc fragment is shown in SEQ ID NO: 12, and,
(b) treating the Fc variant with β-1,4-galactosyltransferase (β1,4-GT) and/or α-2,6-sialyltransferase (α2,6-ST), preferably the β1,4-GT and/or α2,6-ST are from mammalian, more preferably from human.

16. The method of claim 15, wherein the mutation in step (a) is selected from the group consisting of M252Y/S254T/T256E/H433K/N434F, V264A, Δ446G, Δ447K, V262E, T307P/L309Q/Q311R, V264E, ΔE293, ΔE294, M252Y/S254T/T256E, N434A, N434W, N434Y, F241A, F243A, M428L/N434S, L309D/Q311H/N434S, M252Y/T256D, T250Q/M428L, T307Q/N434A, M252Y/V308P/N434Y, H285D/T307Q/A378V, T256D/T307Q, T256D/T307W,and Y300A.

17. An isolated nucleic acid encoding the Fc variant of any one of claims 1-12 or the protein of claim 13.

18. A vector comprising the nucleic acid of claim 17.

19. A host cell comprising the nucleic acid of claim 17 or the vector of claim 18.

20. A method for preparing the Fc variant of any one of claims 1-12, comprising the following steps:
(i) culturing the host cell of claim 19 in a culture medium under conditions that allow the expression of the Fc variant; and,
(ii) optionally, purifying the Fc variant from cultured cells or the culture medium of cells.

21. Use of the Fc variant of any one of claims 1-12, the protein of claim 13, or the composition or kit of claim 14 in the manufacture of a drug for reducing the level of IgG in the blood.

22. Use of the Fc variant of any one of claims 1-12, the protein of claim 13 or the composition or kit of claim 14 in the manufacture of a drug for the treatment of an antibody-mediated disorder, preferably the antibody-mediated disorder is an autoimmune disease or hyperglobulinemia.

23. The use of claim 22, wherein the autoimmune disease is selected from the group consisting of Addison's disease, alopecia areata, ankylosing spondylitis, antiphospholipid syndrome, aplastic anemia, anti-GBM glomerulonephritis, anti-NMDAR encephalitis, antiphospholipidemic syndrome, autoimmune gastritis, autoimmune hearing loss, autoimmune hemolytic anemia, autoimmune hepatitis, autoimmune hypoparathyroidism, autoimmune hypophysitis, autoimmune inner ear disease, autoimmune lymphoproliferative syndrome, autoimmune myocarditis, autoimmune oophoritis, autoimmune bullous skin disease, autoimmune orchitis, autoimmune polyendocrinopathy, Behcet's disease, bullous pemphigoid, cardiomyopathy, inflammatory demyelinating polyneuropathy, acute motor axonal neuropathy, Churg-Strauss syndrome, abdominal diseases, autoimmune urticaria, Crohn's disease, CREST syndrome, celiac disease, Degos's disease, anti-neutrophil cytoplasmic antibody associated vascular inflammation, autoimmune neutropenia, acquired epidermolysis bullosa, essential mixed cryoglobulinemia, giant cell arteritis, glomerulonephritis, Goodpasture's syndrome, Graves' disease, Guillain-Barre syndrome, Hashimoto's thyroiditis, primary immunologic thrombocytopenic purpura, inflammatory bowel disease, Kawasaki disease, Ménière's syndrome, mixed connective tissue disease, Mooren ulcer, rheumatoid arthritis, multiple sclerosis, myasthenia gravis, stiffman syndrome, complete congenital heart block, acquired epidermolysis bullosa, pemphigus foliaceus, pemphigus vulgaris, pernicious anemia, polyarteritis nodosa, types of polyglandular autoimmune syndrome, primary biliary cirrhosis, types of polyglandular autoimmune syndrome, types of polyglandular autoimmune syndrome, polymyositis/dermatomyositis, psoriasis, psoriatic arthritis, Raynaud's syndrome, Reiter's syndrome, rheumatic heart disease, hemophilia-acquired FVIII deficiency, Lambert-Eaton myasthenia syndrome, multiple myeloma, sarcoidosis, collagen thesaurismosis, Sjogren's syndrome, subacute thyroiditis, sympathetic ophthalmia, systemic lupus erythematosus, Takayasu's arteritis, Sjögren's syndrome, type I diabetes, vitiligo, Vogt-Koyanagi-Harada syndrome or Wegener's granulomatosis, acute asthma or chronic asthma, organ transplant rejection, primary progressive multiple sclerosis, systemic sclerosis, serological disease, and immune complex hypersensitivity; wherein the immune complex is an immune complex formed by an anti-drug antibody and a drug;
preferably, the autoimmune disease is selected from the group consisting of anti-glomerular basement membrane antibody disease (anti-GBM antibody disease), pathogenic IgG antibody-mediated thrombotic thrombocytopenia, Guillain-Barre syndrome (GBS), autoimmune encephalitis (AE), neuromyelitis optica lineage disease (NMOSD), atypical hemolytic uremic syndrome (aHUS), antibody-mediated rejection after organ transplantation (AMR), myasthenia gravis (MG); wherein the pathogenic IgG antibody mediated thrombotic thrombocytopenia is preferably thrombotic thrombocytopenic purpura (TTP), heparin induced thrombocytopenia (HIT), vaccine induced immune thrombotic thrombocytopenia (VITT).

24. The use of claim 22, wherein the antibody-mediated disorder is treatable using intravenous immunoglobulin (IVIG), subcutaneous immunoglobulin (SCIG), plasmapheresis, and/or immunoadsorption.

25. The use of claim 22, wherein the hyperglobulin is produced by a leukocyte selected from the group consisting of B cells and abnormal B cells; the globulin comprises a gamma globulin; the hyperglobulinemia is selected from the group consisting of primary monoclonal gammaglobulinemia, connective tissue disease, liver disease, infectious disease, sarcoidosis, myasthenia gravis, Hodgkin's disease, Behcet's disease, nephritis, allergic purpura, immune (or idiopathic) thrombocytopenia, malignant monoclonal gammaglobulinemia (such as multiple myeloma, heavy chain disease, malignant lymphoma, chronic lymphocytic leukemia, macroglobulinemia, etc.), secondary monoclonal gammaglobulinemia (such as non-lymphoreticular tumor, monocytic leukemia, cryoglobulinemia, etc.), benign M-proteinemia, monoclonal gammopathy of unknown significance (MGUS), Waldenstrom's macroglobulinemia, AL-type amyloidosis, solitary plasmacytoma (bone or extraosseous), POMES syndrome, reactive plasmacytosis, osteolytic lesions of metastatic cancer, plasmoblastic lymphoma, and monoclonal immunoglobulin-associated renal damage (MGRS); preferrably, the hyperglobulinemia is multiple myeloma.

26. Use of the Fc variant of any one of claims 1-12, the protein of claim 13, or the composition or kit of claim 14 in the manufacture of a drug for preventing and/or treating autoantibody-mediated organ rejection after solid organ transplantation in a subject.

27. The use of claim 26, wherein the organ is selected from the group consisting of the kidney, pancreatic islet, pancreas, heart, liver, lung, or small intestine.

28. Use of the Fc variant of any one of claims 1-12, the protein of claim 13, or the composition or kit of claim 14 in the manufacture of a drug for gene therapy.

29. Use of the Fc variant of any one of claims 1-12, the protein of claim 13 or the composition or kit of claim 14 in the preparation of a drug for the clearance of a neutralizing antibody of a pre-existing antiviral vector in vivo prior to a viral vector-based gene therapy.

30. Use of the Fc variant of any one of claims 1-12, the protein of claim 13, or the composition or kit of claim 14 in the manufacture of a drug for the clearance of autoantibodies in a subject to render an Fc-containing agent more therapeutically effective.

31. Use of the Fc variant of any one of claims 1-12, the protein of claim 13, or the composition or kit of claim 14 in the manufacture of a drug for reducing the serum level of an Fc-containing agent in a subject to whom the Fc-containing agent has been administered.

32. The use of claim 30 or 31, wherein the Fc-containing agent is a therapeutic or diagnostic agent, preferably the Fc-containing agent is an antibody, Fc fusion protein antibody drug conjugate.

33. The use of any one of claims 21-32, wherein the Fc variant is simultaneously or sequentially administered to the subject along with another therapeutic agent, wherein the other therapeutic agent is an anti-inflammatory agent, a leukocyte depleting agent, or a B-cell depleting agent; the B-cell depleting agent is preferably used as an antibody for tumor treatment, more preferably the antibody specifically binds to CD10, CD19, CD20, CD21, CD22, CD23, CD24, CD37, CD53, CD70, CD72, CD74, CD75, CD77, CD79a, CD79b, CD80, CD81, CD82, CD83, CD84, CD3, CD11a, CD14, CD25, CD28, CD30, CD33, CD36, CD38, CD40, CD44, CD52, CD55, CD59, CD56, CD103, CD134, CD137, CD138, CD152, CD3, IL-1p, IL-2R, IL-6, IL-12, IL-23, C5, BAFF, BLyS, BCMA, CXCR-4, ICAM-1, SLAMF7/CS1, TNFα, IgE, CD85, or CD86.

34. The use of claim 33, wherein the additional therapeutic agent is selected from the group consisting of rituximab, daclizumab, basiliximab, muromonab-CD3, infliximab, adalimumab, omalizumab, efalizumab, natalizumab, tocilizumab, eculizumab, golimumab, canakinumab, ustekinumab, belimumab, daratumumab, Isatuximab, or a combination thereof.
